# EUROPEAN PATENT SPECIFICATION

(11) **EP 2 651 486 B1**
(45) Date of publication and mention of the grant of the patent: **13.04.2016**
(21) Application number: 11848030.0
(22) Date of filing: 05.12.2011
(51) Int. Cl.: A61F 9/007

(54) **INFUSION SLEEVE WITH MULTIPLE MATERIAL LAYERS**
INFUSIONSHÜLSE MIT MEHREREN MATERIALSCHICHTEN
MANCHON D'INFUSION AVEC DE MULTIPLES COUCHES DE MATÉRIAU

(30) Priority: 15.12.2010 US 423183 P
(43) Date of publication of application: 23.10.2013
(73) Proprietor: Alcon Research, Ltd., Fort Worth, Texas 76134 (US)
(72) Inventor: LIAO, Grace Chuang, Irvine, California 92620 (US); HONG, Karen T., Corona, California 92882 (US)
(74) Representative: Hanna, Peter William Derek
(86) International application number: PCT/US2011/063242
(87) International publication number: WO 2012/082425

(56) References cited:
- WO-A1-99/21513
- WO-A2-98/25542
- US-A- 5 188 589
- US-A- 5 505 693
- US-A- 5 505 693
- US-A- 5 807 310
- US-A1- 2006 100 653
- US-B1- 6 280 449
- US-B2- 7 445 596

## Description

### TECHNICAL FIELD

The present disclosure generally relates to the field of phacoemulsification, and more specifically the field of infusion sleeves.

### BACKGROUND

Typical phacoemulsification ultrasonic surgical devices consist of an ultrasonically driven handpiece with an attached cutting tip and irrigating sleeve, also known as an infusion sleeve, with an electronic control module. The handpiece generally includes an irrigation line, aspiration line and a power cord, all of which are generally interconnected to, and controlled by, the control module. In use, the cutting tip and attached infusion sleeve are inserted into a small incision in a cornea, or other location. The control module varies a power level in the handpiece to ultrasonically vibrate the cutting tip, while providing irrigation between the infusion sleeve and cutting tip. The control module also provides a vacuum through a port in the cutting tip.

The infusion sleeve is the only part, other than the cutting tip, that comes into contact with a patient. Thus, the infusion sleeve's operability is extremely important for the overall performance of the phacoemulsification equipment. One concern in phacoemulsification surgical procedures is the problem of heat build-up in the cutting tip. Wound pressure on the infusion sleeve walls compresses the walls and causes both reduced fluid flow to and from the cutting tip and heat-producing frictional contact between the vibrating cutting tip and the walls of the infusion sleeve. Thus, as cooling fluid flow is diminished, frictional heat increases without a means to dissipate the heat. The heat build-up can cause scleral or corneal burns very quickly. This problem becomes an increasing concern when higher frequency (i.e. higher energy) vibrations are used. Passively, corneal burns may be mitigated by a surgeon through appropriate adjustments of a combination of operating parameters, such as the amount of phaco power delivered and the volume of irrigation/aspiration flow. Additionally, some prior art handpiece assemblies (or probes) generally have relied solely on a textured inner sleeve wall and the flow of the irrigant between the cutting tip and the sleeve and the flow of aspirated material into the cutting tip bore to cool the cutting tip. However, a viscoelastic material injected into the anterior ocular chamber during a typical phacoemulsification procedure resists the flow of the irrigant out of the sleeve and is highly resistant to aspiration flow into the cutting tip bore. Therefore, the flow of aspiration and irrigation fluids into and out of the eye can be momentarily occluded whenever the cutting tip and sleeve come into contact with the viscoelastic material. This momentary occlusion can result in sudden cutting tip overheating and resultant scleral and/or corneal lesions. Thus, because cutting tip overheating occurs very rapidly (within 1 to 3 seconds), even short term exposure to such overheating can cause injury to delicate eye tissue.

Other prior art attempts to prevent heat transmission methods have been employed through the use of "Mackool tips." A Mackool tip is a two part infusion sleeve having an inner rigid sleeve that is inserted into and surrounded by an outer malleable sleeve. The inner rigid sleeve is considered as part of the Mackool phaco tip and sits loosely on a main shaft portion of the Mackool phaco tip. The purpose of the inner rigid sleeve is to decouple the vibrating needle from the infusion sleeve. The inclusion of the inner rigid sleeve results in a much more dramatic reduction of heat.

While the Mackool tip has been proven effective in reducing heat generation, there remains room for improvement. Specifically, the need arises for an infusion sleeve that eliminates the use of the separate inner rigid sleeve, while still providing additional heat reducing properties with reduced friction in a single piece infusion sleeve.

### BRIEF SUMMARY

The present invention provides a phacoemulsification sleeve in accordance with claims which follow. A phacoemulsification system is disclosed. The system includes a phacoemulsification handpiece and selectively detachable infusion sleeve. The infusion sleeve may include a single body portion defined by a first open end and a second open end and having a hollow interior portion therein. The body portion may include multiple layers with at least one inner layer and at least one outer layer, such that the layers are constructed of separate and distinct materials.

US 5,505,693A (Mackool) and WO99/21513A are representative of the present state of the art.

### BRIEF DESCRIPTION OF THE DRAWINGS

Exemplary embodiments of the present disclosure will now be described by way of example in greater detail with reference to the attached figures, in which:
FIG. 1 is a perspective view of an exemplary phacoemulsification handpiece assembly;
FIG. 2 is an enlarged perspective view of an exemplary multi-layer infusion sleeve engaged with the phacoemulsification handpiece of FIG. 1;
FIG. 3 is a perspective cut-away view of the exemplary multi-layer infusion sleeve of FIG. 2, illustrating internal areas of the infusion sleeve; and
FIG. 4 is an enlarged perspective cut-away view of the exemplary multilayer infusion sleeve of FIG. 3, illustrating the multiple layers of the infusion sleeve;
FIG. 5 is an enlarged cut-away view of a portion of the exemplary multilayer infusion sleeve with raised protrusions for friction relieving elements;
FIG. 6 is an enlarged cut-away view of a portion of the exemplary multilayer infusion sleeve with recessed valleys for friction relieving elements;
FIG. 7 is an enlarged cut-away view of a portion of the exemplary multilayer infusion sleeve with a low friction coating for the friction relieving element.

### DETAILED DESCRIPTION

Referring now to the discussion that follows and also to the drawings, illustrative approaches to the disclosed devices are shown in detail. Although the drawings represent some possible approaches, the drawings are not necessarily to scale and certain features may be exaggerated, removed, or partially sectioned to better illustrate and explain the present disclosure. Further the descriptions set forth herein are not intended to be exhaustive or otherwise limit or restrict the claims to the precise forms and configurations shown in the drawings and disclosed in the following detailed description.

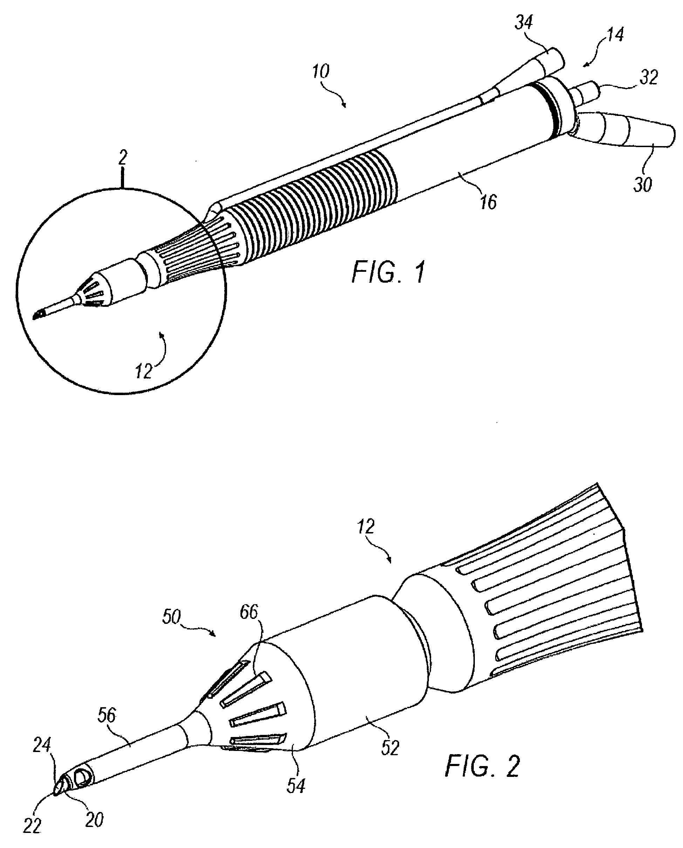

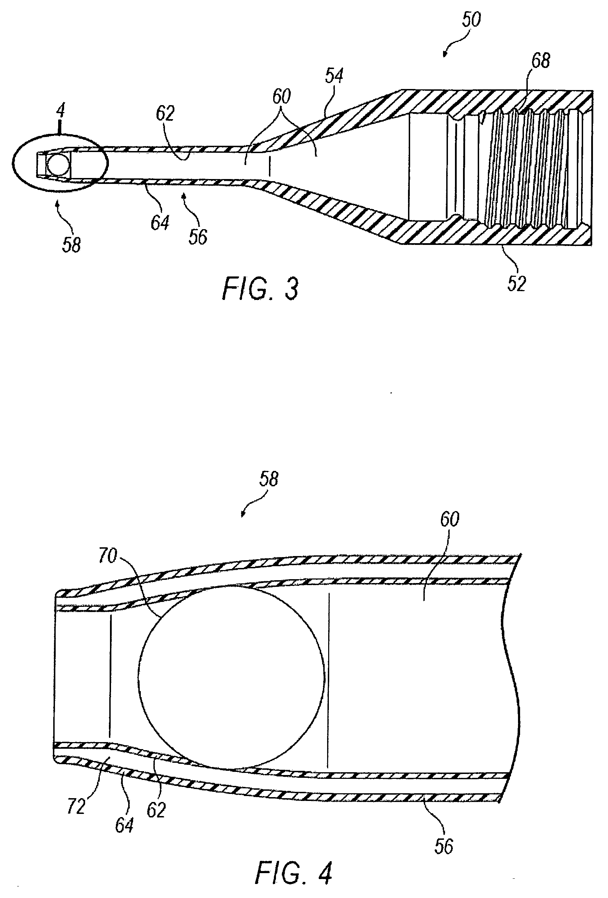

Referring to FIGS. 1 and 2, an exemplary phacoemulsification device 10 is illustrated. The phacoemulsification device 10 is defined by a distal end 12 and a proximal end 14, with a hollow handpiece body portion 16 spanning therebetween. The body portion 16 may be rotatively interconnected at the distal end 12 with an irrigation infusion sleeve 50 (best seen in FIG. 2). The body portion 16 may generally include an ultrasonically driven internal cannula 20 that extends internally from the proximal end 14 to an integrated cutting tip 22 at the distal end 12. The internal cannula 20 may be in communication with a power cord 30, which controls power for ultrasonic vibrations of the cutting tip 22 and an aspiration line 32 for removal of emulsified cataract tissue or aspirant. Additionally, an irrigation line 34 may be fluidly connected to a tube 56, having a hollow area 60, (best seen in FIGS 3 and 4) between an inner surface 62 of the infusion sleeve 50 and an outer surface of the cannula 20.

As can be seen in FIGS. 1 and 2, the infusion sleeve 50 may be attached directly fixed to the distal end 12 of the body portion 16 of the phacoemulsification device 10. The infusion sleeve 50 may generally include a main body or base section 52 that extends from a connection at the distal end 12 of the body portion 16 to a conical nose section 54 that tapers down from the base section 52 to an elongated tube section 56. The infusion sleeve 50 generally includes the hollow area 60 that extends interiorly from the base section 52 to a tapered tip 58. The tapered tip 58 may include one or more fluid ports 70 that fluidly connect the inner hollow area 60 with an incision in the corneal area. Additionally, the infusion sleeve 50 may also include protrusions 66 (best seen in FIG. 2) extending from an outer surface 64 of the infusion sleeve 50 at the conical nose section 54 or the base section 52. The protrusions 66 may provide rigidity and added grip for handling the phacoemulsification device 10.

Turning to FIGS. 3 and 4, an exemplary fusion sleeve 50 is illustrated in cross-section. The infusion sleeve 50, as illustrated, includes threads 68 for rotatively connecting the infusion sleeve 50 to the body portion 16. However, other connecting elements may be used, such as, but not limited to, tongue and groove arrangements, latching male and female tabs, twist lock male tabs and corresponding female receptacles (i.e., luer-type arrangements) and other known elements that provide a sealing connection between the infusion sleeve 50 and the body portion 16. With further reference to FIG. 3, the inner hollow area 60 may be seen as it transitions from the threaded portion 68 and tapers down to the tapered tip 58, where the fluid ports 70 are positioned. Specifically, as discussed above, the fluid ports 70 may be positioned about an outer periphery of the tapered tip 58. The fluid ports 70 may be of any size and of any shape depending on the application. As illustrated, the fluid ports 70 are circular apertures that are positioned on opposing sides.

As illustrated in FIGS. 3 and 4 and discussed above, the infusion sleeve 50 includes the outer surface layer 64 and the inner surface layer 62. In the exemplary arrangement, the outer surface layer 64 extends the length of the infusion sleeve 50 and may be made with materials that provide a minimization of heat and motion transfer. Generally, such outer surface layer 64 materials may include, but are not limited to, a silicone, a polyimide, a polyethylene and an acetal. Each material may have a specific equivalent durometer range (discussed below) with a general thermal conductivity in the range of 0.1 - 0.9 W/(m*°K). Specifically, silicone may have a durometer ranging from 30 - 80 Shore A, polyimide may have a durometer ranging from 65 - 92 Shore D, polyethylene may have a durometer ranging from 45 - 53 Shore D and acetal may have a durometer ranging from 70 - 88 Shore D. However, regardless of which material is used the durometer of the outer layer material will be lower than the durometer of the inner layer material, as the inner layer is more rigid than the outer layer. Specifically, the outer surface layer 64 may be used to minimize the heat and motion transfer that may be transmitted to the cornea, which may help reduce the likelihood of corneal bums and mechanical trauma during a phacoemulsification surgery procedure. The softer material used may also provide better sealing about the incision, thereby reducing any incisional leakage that may occur.

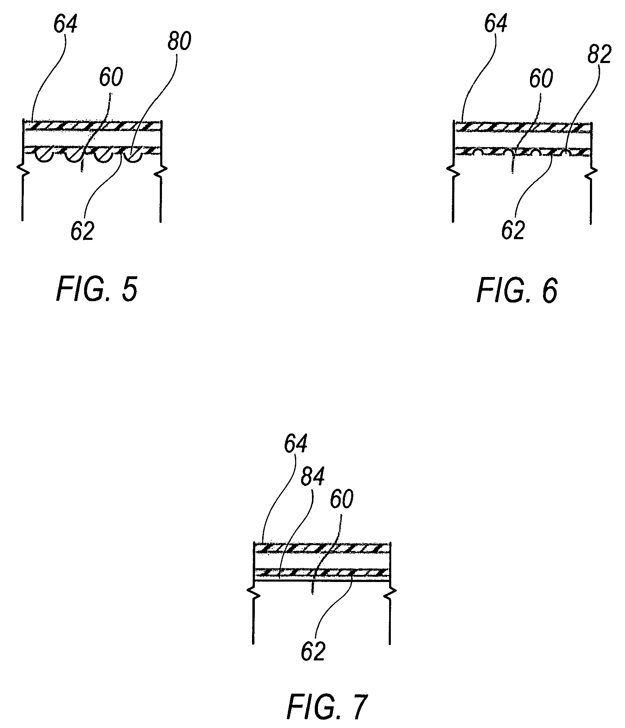

Additionally, the inner surface layer 62 may be made with materials providing a relatively stiff surface that may have a relatively low coefficient of friction such that the surface friction is minimized against the vibrating cutting tip 22. Generally, such inner surface layer 62 materials may include, but are not limited to an injection-moldable polyimide, and an acetal. These materials may typically have a coefficient of friction value approximately in a range of 0.1 to 0.3. The inner surface layer 62 may also include mechanical features, such as, but not limited to, bumps 80 (see FIG. 5), ribs, ridges, recessed valleys 82 (see FIG. 6), or other such friction relieving elements causing a random disruption in the inner surface layer 62. The mechanical features may be used in conjunction with specific manufacturing materials that are different than that of the outer surface 64. Additionally, the inner surface layer 62 may include (either separately or in combination with one or more other friction relieving elements) a low-friction coating 84 (see FIG. 7) as a friction relieving element. The coating may be, but is not limited to a parylene N or other types of parylene coatings and a FluoroBond® LSR (Orion Industries) or other known fluoropolymer coatings generally having a coefficient of friction approximately in the range of 0.1 to 0.3. Depending on the application, the layer materials, mechanical features and coatings may be used simultaneously or each may be used individually. In some embodiments, at least two different layers are used and the inner layer material is more rigid than the outer layer material.

Multiple layers may be used to simultaneously impart additional heat and friction-reducing properties, as opposed to the previous single material designs and previous multi-piece designs. In one exemplary arrangement, the two layers 62, 64 may be created using multi-layer injection molding or overmolding processes. Merely by way of example, multi-component, multi-shot and over-molding process may be employed to create a multiple layered infusion sleeve in a unitary construction.

Multi-component molding may be further subdivided into co-injection, bi-injection and interval injection. Co-injection molding involves making sequential injections into the same mold with one material as the core and one as the skin. It may also be known as sandwich molding because the core is fully encapsulated. Bi-injection molding is the simultaneous injection of different materials through different gates. Interval injection molding, also known as marbling, is the simultaneous injection of different materials through different gates giving limited mixing.

Multi-shot molding describes any process where distinct material shots are applied to produce the final component. This includes transfer molding, injection molding, core back molding and rotating tool molding. Where multiple slugs of different materials may be injected into the mold to create the multiple layered product.

Over-molding includes both insert molding and lost core molding, the latter produces hollow parts. Over-molding allows the desired part to be preformed and then reinserted into a mold for an additional layer of material to be applied to the original part.

In use, the infusion sleeve threads 68 may be rotatively engaged with corresponding threads in the body portion 16 to lock the two pieces as one unit. Further assembly includes attaching the aspiration line 32, irrigation line 34 and power cord 30 to the control module to create a phacoemulsification assembly. It should be known that the infusion sleeve 50 may be of various diameters and lengths to accommodate specific phacoemulsification cannula 20 and body portions 16. Infusion sleeve 50 selection may include selecting a sleeve to allow the cutting tip 22 to extend a predetermined distance from the end of the infusion sleeve 50 to allow for proper alignment of the cutting tip 22 extending past the fluid ports 70. This may allow the cutting tip 22 to provide enough ultrasonic vibration to breakup or emulsify the cataract and draw in just the cataract tissue and not draw additional liquid from the incision. This predetermined distance may also help to eliminate any irrigant from being drawn directly from the hollow area 60 within the infusion sleeve 50.

Once the assembly is created, an operator may activate the control module to cause the phacoemulsification device cannula 20 to vibrate ultrasonically, and these vibrations are transmitted along to the cutting tip 22 where the vibrations are used to fracture or emulsify a cataract or other phaco-type tissue. A vacuum source may be activated to draw the emulsified tissue or aspirant through a central lumen 24 in the cannula 20 (illustrated in FIGS. 1 and 2) starting at the cutting tip 22 and extending through the body portion 16 to a flexible aspiration line 32 that extends to the control module. An irrigant source supplies an irrigant such as saline solution under pressure through irrigation line 34 and that extends along the body portion 16 to the hollow area 60 in the infusion sleeve 50 where the irrigant is forced to migrate along the inner surface layer 62 in the tube 56 between the tube 56 and the cannula 20. The tube 56 may be dimensioned to fit securely around the cannula 20, which allows the irrigant to exit the infusion sleeve 50 through the fluid ports 70 to provide lubrication to the incision area.

As discussed above, the interaction between the infusion sleeve 50 and the cannula 20 may result in overheating and unwanted vibration. Therefore the exemplary infusion sleeve 50 may be constructed with an additional layer 72 of material, as illustrated in FIG. 4. The additional layer 72 may provide additional friction-relieving properties, additional heat resistance properties, or a combination thereof, as discussed above regarding the materials used in producing the multi-layered infusion sleeve 50. These additional requirements may depend upon the predetermined and desired applications. It should be known that when the mechanical elements are used, the amount of frictional contact area between the cannula 20 and the inner surface 62 may be reduced/minimized to lower the frictional heat created by the vibrations. In addition, when mechanical elements are used, an additional lubricity may be retained due to material selection or by retaining the irrigant within the recesses of the elements to continuously supply a small amount of irrigant even if the main flow of irrigant is interrupted, thereby continuously bathing the cannula 20 and ultimately the cutting tip 22 with the lubricating irrigant.

The embodiments aspects and examples which do not fall within the scope of the claims are provided for illustrative purpose only and do not form part of the present invention

The invention is defined in the claims as follows.

## Claims

1. A phacoemulsification infusion sleeve (50), comprising:
a main body portion (52) defined by a first open end that extends to a conical nose section (54) that tapers down from the main body portion (52) to an elongated tube section (56) having a tapered tip (58) that includes one or more fluid ports (70) and terminates at a distal second open end, the sleeve configured to couple to a phacoemulsification device (10), wherein the sleeve comprises hollow interior portion (60) therein extending between the first open end and the second open end; and
wherein the sleeve includes at least one outer layer (64) and at least one inner layer (62),
wherein the at least one outer layer and the at least one inner layer form a unitary construction, wherein the at least one outer layer (64) is constructed of a first material and the at least one inner layer (62) is constructed of a second material, and wherein the second material is more rigid than the first material; **characterized in that**:
the at least one inner layer (62) includes at least one friction relieving mechanical element, such as bumps 80, ribs, ridges, recessed vall eys 82, causing a random disruption in the inner airface layer 62.

2. The phacoemulsification infusion sleeve (50) of claim 1, wherein at least one additional layer (72) is positioned between the inner layer (62) and the outer layer (64).

3. The phacoemulsification infusion sleeve (50) of claim 2, wherein the first material has a durometer range of at least one of approximately 30-50 Shore A and approximately 50-80 Shore D.

4. The phacoemulsification infusion sleeve of claim 1, wherein the at least one friction relieving mechanical feature comprises at least one of a raised protrusion (80) and a recessed valley (82).

5. The phacoemulsification infusion sleeve of claim 1, further comprising a low friction coating (84) as a friction relieving element.

6. The phacoemulsification infusion sleeve of claim 5, wherein the low friction (84) coating is at least one of a parylene coating and a fluoropolymer coating.

7. The phacoemulsification infusion sleeve of claim 1, wherein the second material has a coefficient of friction value in a range of approximately 0.1 to 0.3.

8. The phacoemulsification infusion sleeve (50) of claim 1, wherein the second material is biocompatible material and is constructed from one of a polyimide and an acetal.

9. The phacoemulsification infusion sleeve (50) of claim 1, wherein the first material has a durometer range of at least one of approximately 30-80 Shore A and approximately 45-92 Shore D.

10. The phacoemulsification infusion sleeve (50) of claim 1, wherein the first material has a general thermal conductivity in a range of approximately 0.1- 0.9 Watts per meter Kelvin.

11. The phacoemulsification infusion sleeve (50) of claim 1, wherein the first material is a biocompatible material and is constructed from one of a silicone, a polyimide, a polyethylene and an acetal to minimize at least one of heat transfer and motion transfer.

12. The phacoemulsification infusion sleeve (50) of claim 2, wherein the at least one additional layer (72) is constructed from a material having at least one of heat minimizing properties, motion transfer minimizing properties and friction reducing properties.

13. The phacoemulsification infusion sleeve (50) of claim 13, wherein the at least one additional layer (72) is a biocompatible material and is constructed from one of a silicone, a polyimide, a polyethylene and an acetal.

14. The phacoemulsification infusion sleeve (50) of claim 1, wherein the at least one outer layer (64) and the at least one inner layer (62) of the body portion are molded together to form the unitary construction.

15. The phacoemulsification infusion sleeve (50) of claim 14, wherein the at least one outer layer (64) and at least one inner layer (62) form a co-injected molded body.

16. The phacoemulsification infusion sleeve (50) of claim 14, wherein the at least one outer layer (64) is overmolded onto the at least one inner layer (62).

17. The phacoemulsification infusion sleeve (50) of claim 14, wherein the at least one outer layer (64) and at least one inner layer (62) form a bi-injected molded body.

18. The phacoemulsification infusion sleeve (50) of claim 4, wherein the at least one friction relieving mechanical feature comprises bumps (80), ribs, ridges, or recessed valleys (82).

19. The phacoemulsification infusion sleeve (50) of claim 1, wherein the at least one outer layer (64) and at least one inner layer (62) extend from the first open end to the distal second open end.

## Patentansprüche

1. Infusionshülse (50) für Phakoemulsifikation, umfassend:
einen Hauptgehäuseabschnitt (52), der definiert wird von einem ersten offenen Ende, das sich zu einem konischen Nasenabschnitt (54) erstreckt, welcher sich von dem Hauptgehäuseabschnitt (52) zu einem länglichen Rohrabschnitt (56) mit einer sich verjüngenden Spitze (58), die einen oder mehrere Fluidanschlüsse (70) umfasst, verjüngt, und der an einem distalen zweiten offenen Ende endet, wobei die Hülse dazu konfiguriert ist, sich mit einer Phakoemulsifikationsvorrichtung (10) zu koppeln, wobei die Hülse einen hohlen Innenabschnitt (60) umfasst, der sich darin zwischen dem ersten offenen Ende und dem zweiten offenen Ende erstreckt; und
wobei die Hülse wenigstens eine äußere Schicht (64) und wenigstens eine innere Schicht (62) umfasst,
wobei die wenigstens eine äußere Schicht und die wenigstens eine innere Schicht eine einheitliche Konstruktion bilden, wobei die wenigstens eine äußere Schicht (64) aus einem ersten Material hergestellt ist, und die wenigstens eine innere Schicht (62) aus einem zweiten Material hergestellt ist, und wobei das zweite Material steifer als das erste Material ist;
**dadurch gekennzeichnet, dass**
die wenigstens eine innere Schicht (62) wenigstens ein reibungsminderndes mechanisches Element wie z. B. erhöhte Fortsätze (80), Rippen, Grate oder Vertiefungen (82) umfasst, das zufällige Unterbrechungen in der inneren Oberflächenschicht (62) verursacht.

2. Infusionshülse (50) für Phakoemulsifikation gemäß Anspruch 1, wobei wenigstens eine zusätzliche Schicht (72) zwischen der inneren Schicht (62) und der äußeren Schicht (64) angeordnet ist.

3. Infusionshülse (50) für Phakoemulsifikation gemäß Anspruch 2, wobei das erste Material einen Durometer-Bereich von etwa 30-50 Shore A und/oder etwa 50-80 Shore D aufweist.

4. Infusionshülse für Phakoemulsifikation gemäß Anspruch 1, wobei das wenigstens eine reibungsmindernde mechanische Element einen erhöhten Fortsatz (80) und/oder eine Vertiefung (82) umfasst.

5. Infusionshülse für Phakoemulsifikation gemäß Anspruch 1, weiterhin umfassend eine reibungsarme Beschichtung (84) als reibungsminderndes Element.

6. Infusionshülse für Phakoemulsifikation gemäß Anspruch 5, wobei es sich bei der reibungsarmen Beschichtung (84) um eine Parylenbeschichtung und/oder eine Fluoropolymerbeschichtung handelt.

7. Infusionshülse für Phakoemulsifikation gemäß Anspruch 5, wobei das zweite Material einen Reibungskoeffizientenwert in einem Bereich von etwa 0,1 bis 0,3 aufweist.

8. Infusionshülse (50) für Phakoemulsifikation gemäß Anspruch 1, wobei es sich bei dem zweiten Material um ein biokompatibles Material handelt, das aus Polyimid oder Acetal hergestellt ist.

9. Infusionshülse (50) für Phakoemulsifikation gemäß Anspruch 1, wobei das erste Material einen Durometer-Bereich von etwa 30-80 Shore A und/oder etwa 45-92 Shore D aufweist.

10. Infusionshülse (50) für Phakoemulsifikation gemäß Anspruch 1, wobei das erste Material eine allgemeine Wärmeleitfähigkeit in einem Bereich von etwa 0,1 bis 0,9 Watt pro Meter Kelvin aufweist.

11. Infusionshülse (50) für Phakoemulsifikation gemäß Anspruch 1, wobei es sich bei dem ersten Material um ein biokompatibles Material handelt, das aus Silikon, Polyimid, Polyethylen und/oder Acetal hergestellt ist, um eine Wärmeübertragung und/oder eine Bewegungsübertragung zu reduzieren.

12. Infusionshülse (50) für Phakoemulsifikation gemäß Anspruch 2, wobei die wenigstens eine zusätzliche Schicht (72) aus einem Material hergestellt ist, das Eigenschaften zur Wärmereduzierung, zur Bewegungsübertragungsreduzierung und/oder zur Reibungsreduzierung aufweist.

13. Infusionshülse (50) für Phakoemulsifikation gemäß Anspruch 13, wobei es sich bei der wenigstens einen zusätzlichen Schicht (72) um ein biokompatibles Material handelt, das aus Silikon, Polyimid, Polyethylen oder Acetal hergestellt ist.

14. Infusionshülse (50) für Phakoemulsifikation gemäß Anspruch 1, wobei die wenigstens eine äußere Schicht (64) und die wenigstens eine innere Schicht (62) des Gehäuseabschnitts zusammengegossen werden, um die einheitliche Konstruktion bilden.

15. Infusionshülse (50) für Phakoemulsifikation gemäß Anspruch 14, wobei die wenigstens eine äußere Schicht (64) und die wenigstens eine innere Schicht (62) einen co-injizierten Formkörper bilden.

16. Infusionshülse (50) für Phakoemulsifikation gemäß Anspruch 14, wobei die wenigstens eine äußere Schicht (62) über die wenigstens eine innere Schicht (62) gegossen wird.

17. Infusionshülse (50) für Phakoemulsifikation gemäß Anspruch 14, wobei die wenigstens eine äußere Schicht (64) und die wenigstens eine innere Schicht (62) einen bi-injizierten Formkörper bilden.

18. Infusionshülse (50) für Phakoemulsifikation gemäß Anspruch 4, wobei das wenigstens eine reibungsmindernde mechanische Element erhöhte Fortsätze (80), Rippen, Grate oder Vertiefungen (82) umfasst.

19. Infusionshülse (50) für Phakoemulsifikation gemäß Anspruch 1, wobei die wenigstens eine äußere Schicht (64) und die wenigstens eine innere Schicht (62) sich von dem ersten offenen Ende zu dem distalen zweiten offenen Ende erstrecken.

## Revendications

1. Manchon de perfusion de phacoémulsification (50), comprenant :
une partie de corps principal (52) définie par une première extrémité d'ouverture qui se prolonge vers une section de partie avant conique (54) qui se rétrécit vers le bas à partir de la partie de corps principal (52) vers une section de tube allongée (56) ayant une pointe fuselée (58) qui comprend un ou plusieurs orifices de fluide (70) et se termine au niveau d'une seconde extrémité d'ouverture distale, le manchon étant configuré pour se coupler à un dispositif de phacoémulsification (10), dans lequel le manchon comprend une partie interne creuse (60) se prolongeant entre la première extrémité d'ouverture et la seconde extrémité d'ouverture ; et
dans lequel le manchon comprend au moins une couche externe (64) et au moins une couche interne (62),
dans lequel l'au moins une couche externe et l'au moins une couche interne forment une construction unitaire, où l'au moins une couche externe (64) est constituée d'un premier matériau et l'au moins une couche interne (62) est constituée d'un second matériau, et où le second matériau est plus rigide que le premier matériau ; **caractérisé en ce que** :
l'au moins une couche interne (62) comprend au moins un élément mécanique de dissipation de friction tel que des bosses (80), des nervures, des crêtes, des vallées encastrées (82), provoquant une perturbation aléatoire de la surface de la couche interne (62).

2. Manchon de perfusion de phacoémulsification (50) selon la revendication 1, dans lequel au moins une couche supplémentaire (72) est positionnée entre la couche interne (62) et la couche externe (64).

3. Manchon de perfusion de phacoémulsification (50) selon la revendication 2, dans lequel le premier matériau a une plage de duromètre d'environ 30 à 50 Shore A et/ou d'environ 50 à 80 Shore D.

4. Manchon de perfusion de phacoémulsification selon la revendication 1, dans lequel l'au moins un élément mécanique de dissipation de friction comprend une saillie surélevée (80) et/ou une vallée encastrée (82).

5. Manchon de perfusion de phacoémulsification selon la revendication 1, comprenant en outre un revêtement de faible friction (84) en tant qu'élément de dissipation de friction.

6. Manchon de perfusion de phacoémulsification selon la revendication 5, dans lequel le revêtement de faible friction (84) est un revêtement de parylène et/ou un revêtement de polymère fluoré.

7. Manchon de perfusion de phacoémulsification selon la revendication 1, dans lequel le second matériau a une valeur de coefficient de friction comprise dans la plage allant de 0,1 à 0,3.

8. Manchon de perfusion de phacoémulsification (50) selon la revendication 1, dans lequel le second matériau est un matériau biocompatible et se compose d'un polyimide et/ou d'un acétal.

9. Manchon de perfusion de phacoémulsification (50) selon la revendication 1, dans lequel le premier matériau a une plage de duromètre d'environ 30 à 80 Shore A et/ou d'environ 45 à 92 Shore D.

10. Manchon de perfusion de phacoémulsification (50) selon la revendication 1, dans lequel le premier matériau a une conductivité thermique générale comprise dans la plage allant d'environ 0,1 à 0,9 Watts par mètre kelvin.

11. Manchon de perfusion de phacoémulsification (50) selon la revendication 1, dans lequel le premier matériau est un matériau biocompatible et est constitué d'une silicone, d'un polyimide, d'un polyéthylène et/ou d'un acétal pour minimiser le transfert de chaleur et/ou le transfert de mouvement.

12. Manchon de perfusion de phacoémulsification (50) selon la revendication 2, dans lequel l'au moins une couche supplémentaire (72) est constituée d'un matériau ayant des propriétés minimisant la chaleur, des propriétés minimisant le transfert de mouvement et/ou des propriétés réduisant la friction.

13. Manchon de perfusion de phacoémulsification (50) selon la revendication 13, dans lequel l'au moins une couche supplémentaire (72) est un matériau biocompatible et est constituée d'une silicone, d'un polyimide, d'un polyéthylène et/ou d'un acétal.

14. Manchon de perfusion de phacoémulsification (50) selon la revendication 1, dans lequel l'au moins une couche externe (64) et l'au moins une couche interne (62) de la partie de corps sont moulées ensemble pour former la construction unitaire.

15. Manchon de perfusion de phacoémulsification (50) selon la revendication 14, dans lequel l'au moins une couche externe (64) et l'au moins une couche interne (62) forment un corps moulé co-injecté.

16. Manchon de perfusion de phacoémulsification (50) selon la revendication 14, dans lequel l'au moins une couche externe (64) est surmoulée sur l'au moins une couche interne (62).

17. Manchon de perfusion de phacoémulsification (50) selon la revendication 14, dans lequel l'au moins une couche externe (64) et l'au moins une couche interne (62) forment un corps moulé bi-injecté.

18. Manchon de perfusion de phacoémulsification (50) selon la revendication 4, dans lequel l'au moins un élément mécanique de dissipation de friction comprend des bosses (80), des nervures, des crêtes ou des vallées encastrées (82).

19. Manchon de perfusion de phacoémulsification (50) selon la revendication 1, dans lequel l'au moins une couche externe (64) et l'au moins une couche interne (62) se prolongent de la première extrémité d'ouverture vers la seconde extrémité d'ouverture distale.
